# EUROPÄISCHE PATENTSCHRIFT

(11) **EP 2 468 193 B1**
(45) Veröffentlichungstag und Bekanntmachung des Hinweises auf die Patenterteilung: **30.03.2016**
(21) Anmeldenummer: 11010028.6
(22) Anmeldetag: 21.12.2011
(51) Int. Cl.: A61B 17/04, A61B 17/29, A61B 17/062

(54) **Medizinischer Nadelhalter**
Medical needle holder
Porte-aiguille médical

(30) Priorität: 23.12.2010 DE 102010055807
(43) Veröffentlichungstag der Anmeldung: 27.06.2012
(73) Patentinhaber: Karl Storz GmbH & Co. KG, 78532 Tuttlingen (DE)
(72) Erfinder: Schneider, Sven, 78532 Tuttlingen (DE); Bacher, Uwe, 78532 Tuttlingen (DE)
(74) Vertreter: Hofmeister, Frank

(56) Entgegenhaltungen:
- EP-A2- 1 889 579
- DE-U1- 9 109 097
- US-A- 5 147 373
- US-A1- 2004 225 323
- US-A1- 2010 087 818
- US-B1- 6 269 819
- US-B1- 6 270 508

## Beschreibung

Die Erfindung betrifft einen medizinischen Nadelhalter mit einem Schaft, mit einem am distalen Ende des Schaftes angeordneten, aus zwei Maulteilen bestehenden Werkzeug, wobei ein Maulteil des Werkzeugs gegenüber dem anderen Maulteil des Werkzeugs verschwenkbar ausgebildet ist, sowie mit einer am proximalen Ende des Schaftes angeordneten, mindestens zwei Griffteile aufweisenden Handhabe, wobei das verschwenkbare Maulteil des Werkzeugs über ein verstellbar ausgebildetes Griffteil der Handhabe betätigbar ist.

Gattungsgemäße medizinische Nadelhalter sind in verschiedenen Ausführungsformen bekannt. Die in der Regel sichelförmig gebogenen chirurgischen Nadeln werden von dem Werkzeug ergriffen und klemmend zwischen den Maulteilen des Werkzeugs gehalten. Um die solchermaßen gehaltene Nadel in die richtige Lage zum Vernähen einer Operationsstelle auszurichten, muss der Operateur die Nadel entweder manuell ausrichten oder aber mittels einer weiteren Fasszange in die erforderliche Lage zwischen den Maulteilen des Werkzeugs überführen.

Des weiteren werden durch US6270508 und DE9109097U medizinische Nadelhalter offenbart, welche mittels eines, in Bezug auf ein erstes schwenkbares Maulteil, längsverschiebbaren, zweiten Maulteils eine einfache Positionierung der Nadel im Werkzeug ermöglichen.

Davon ausgehend liegt der Efindung die Aufgabe zugrunde, einen medizinischen Nadelhalter zu schaffen, der eine bessere Positionierung der Nadel im Werkzeug ermöglicht.

Die Lösung dieser Aufgabenstellung ist erfindungsgemäß dadurch gekennzeichnet, dass ein Teilbereich des nicht verschwenkbaren Maulteils des Werkzeugs in Längsrichtung des Schaftes relativ zu dem anderen Maulteil des Werkzeugs verschiebbar ausgebildet ist und dass das Verschieben in Längsrichtung des Schaftes über einen an der Handhabe angeordneten Antrieb erfolgt.

Durch die erfindungsgemäße Ausgestaltung der Maulteile des Werkzeugs, wonach ein Teilbereich mindestens eines Maulteils des Werkzeugs in Längsrichtung des Schaftes relativ zu dem anderen Maulteil des Werkzeugs verschiebbar ausgebildet ist, ist es möglich, die Lage der zwischen den Maulteilen gehaltenen chirurgischen Nadel ohne Zuhilfenahme eines zusätzlichen Werkzeugs oder manuelles Ergreifen auszurichten. Das Verschieben des Teilbereichs eines Maulteils in Längsrichtung des Schaftes erfolgt erfindungsgemäß über einen an der Handhabe angeordneten Antrieb.

Mit einer bevorzugten Ausführungsform der Erfindung wird vorgeschlagen, dass der Antrieb als Stellrad ausgebildet ist, wodurch eine sehr einfache Betätigung mit der Haltehand ermöglicht wird.

Gemäß einer praktischen Ausführungsform der Erfindung wird vorgeschlagen, dass der verschiebbare Teilbereich des Maulteils und der Antrieb über ein Betätigungselement miteinander in Wirkverbindung stehen, das die Rotationsbewegung des Stellrads in eine Längsbewegung des verschiebbaren Teilbereichs des Maulteils überführt.

Zur Übertragung der rein axialen Bewegung zum Betätigen des verschwenkbaren Maulteils wird mit der Erfindung vorgeschlagen, dass das verschwenkbare Maulteil des Werkzeugs und das verstellbar ausgebildete Griffteil der Handhabe über ein Zug-/Druckelement miteinander in Wirkverbindung stehen.

Gemäß einer ersten praktischen Ausführungsform der Erfindung wird vorgeschlagen, dass das Betätigungselement und das Zug-/Druckelement koaxial zueinander im Schaft angeordnet sind. Die koaxiale Anordnung der beiden Bauteile stellt eine platzsparende Ausgestaltungsform dar.

Gemäß einer bevorzugten Ausführungsform der koaxialen Anordnung von Betätigungselement und Zug-/Druckelement wird erfindungsgemäß vorgeschlagen, dass das Betätigungselement als im Schaft angeordnetes Hohlrohr ausgebildet ist, wobei das Stellrad und das Hohlrohr über ein Gewinde miteinander in Wirkverbindung stehen und wobei der verschiebbare Teilbereich des Maulteils das distale Ende des Hohlrohres bildet.

Das Zug-/Druckelement ist bei dieser koaxialen Ausgestaltung vorzugsweise als axial verschiebbar in dem Hohlrohr angeordnete Zug-/Druckstange ausgebildet ist.

Gemäß einer zweiten praktischen Ausführungsform der Erfindung wird vorgeschlagen, dass das Betätigungselement und das Zug-/Druckelement parallel nebeneinander im Schaft angeordnet sind.

Gemäß der parallelen Anordnung von Betätigungselement und das Zug-/Druckelement wird mit der Erfindung vorgeschlagen, dass das Betätigungselement als im Schaft angeordnete Torsionsstange ausgebildet ist, wobei das Stellrad und die Torsionsstange kraftschlüssig miteinander verbunden sind und wobei der verschiebbare Teilbereich des Maulteils das distale Ende der Torsionsstange über ein Gewinde miteinander in Wirkverbindung stehen.

Zum Betätigen des verschwenkbaren Maulteils ist das Zug-/Druckelement bei dieser Ausgestaltungsform vorzugsweise als im Schaft angeordnete Zug-/Druckstange ausgebildet.

Gemäß einer dritten praktischen Ausführungsform der Erfindung wird vorgeschlagen, dass das Betätigungselement und das Zug-/Druckelement als im Schaft angeordnetes einstückiges stangenförmiges Antriebselement ausgebildet sind, über das sowohl die Axialbewegung des verstellbaren Griffteils als auch die Verdrehung des Stellrades übertragbar ist.

Zur Übertragung der Axialbewegung von dem verstellbar ausgebildeten Griffteil der Handhabe auf das verschwenkbare Maulteil des Werkzeugs wird bei dieser einstückigen Ausgestaltungsform vorgeschlagen, dass das Antriebselement proximalseitig über ein kugelförmiges Anschlusselement am verstellbar ausgebildeten Griffteil gelagert ist und distalseitig im Bereich der Lagerung am verschwenkbaren Maulteil in der äußeren Mantelfläche des Antriebselements eine umlaufende Nut ausgebildet ist, in der ein um die Längsachse des Antriebselements drehbarer Ring angeordnet ist, an dem das verschwenkbare Maulteil gelagert ist. Die kugelförmige Ausgestaltung des proximalen Endes des Antriebselements erlaubt eine frei drehbare Lagerung am verstellbaren Griffteil bei gleichzeitiger Spielfreiheit zur Übertragung axialer Bewegungen.

Weiterhin wird mit der Erfindung vorgeschlagen, dass zur Übertragung der Rotationsbewegung des Stellrads in eine Längsbewegung des verschiebbaren Teilbereichs des Maulteils das Antriebselement proximalseitig kraftschlüssig mit dem Stellrad verbunden ist und distalseitig im Bereich der Lagerung am verschiebbaren Teilbereichs des Maulteils kraftschlüssig mit einer koaxial auf dem distalen Ende des Antriebselements gelagerten Hülse in Wirkverbindung steht, die über ein Gewinde mit dem verschiebbaren Teilbereich des Maulteils verbunden ist.

Um die Axialbewegung und die Rotationsbewegung des Antriebselements voneinander zu entkoppeln sind erfindungsgemäß in den Bereichen der kraftschlüssigen Verbindungen des Antriebselements mit dem Stellrad sowie des Antriebselements mit der Hülse Ausgleichsführungen ausgebildet.

Zur Ausbildung der kraftschlüssigen Verbindungen des Antriebselements mit dem Stellrad sowie des Antriebselements mit der Hülse sind erfindungsgemäß Mitnahmestifte im Antriebselement angeordnet, die in das jeweils andere Bauteil Stellrad und Hülse eingreifen.

Schließlich wird mit der Erfindung vorgeschlagen, dass die Ausgleichsführungen als im Stellrad und in der Hülse angeordnete, in Axialrichtung verlaufende Nuten ausgebildet sind, in denen die Mitnahmestifte gelagert sind.

Weitere Merkmale und Vorteile der Erfindung ergeben sich anhand der zugehörigen Zeichnungen, in denen verschiedene Ausführungsbeispiele eines efindungsgemäßen medizinischen Nadelhalters nur beispielhaft dargestellt sind, ohne die Erfindung auf diese Ausführungsbeispiele zu beschränken. In den Zeichnungen zeigt:
- Fig. 1: eine schematische Seitenansicht eines medizinischen Nadelhalters in einer ersten Arbeitsstellung der Maulteile;
- Fig. 2: eine Darstellung gemäß Fig. 1, jedoch die Maulteile in einer zweiten Arbeitsstellung darstellend;
- Fig. 3: eine vergrößerte schematische Ansicht einer ersten Ausführungsform des Details III gemäß Fig. 1;
- Fig. 4: eine vergrößerte schematische Ansicht der ersten Ausführungsform des Details IV gemäß Fig. 1;
- Fig. 5: eine vergrößerte schematische Ansicht einer zweiten Ausführungsform des Details V gemäß Fig. 1;
- Fig. 6: eine vergrößerte schematische Ansicht der zweiten Ausführungsform des Details VI gemäß Fig. 1;
- Fig. 7: eine vergrößerte schematische Ansicht einer dritten Ausführungsform des Details VII gemäß Fig. 1 und
- Fig. 8: eine vergrößerte schematische Ansicht der dritten Ausführungsform des Details VIII gemäß Fig. 1

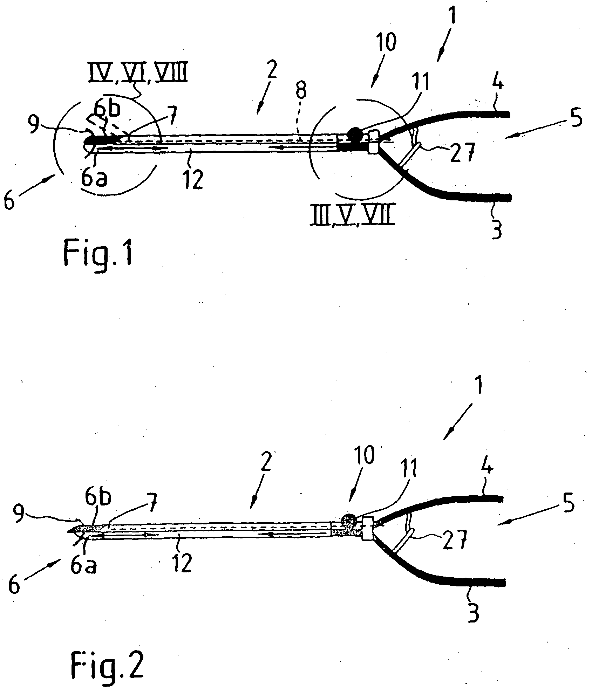

Die Abbildungen Fig. 1 und 2 zeigen die Seitenansichten eines medizinischen Nadelhalters 1.

Der dargestellte medizinische Nadelhalter 1 besteht im Wesentlichen aus einem hohlen Schaft 2, an dessen proximalem Ende eine zwei Griffteilen 3 und 4 aufweisende Handhabe 5 angeordnet ist, wobei bei der dargestellten Ausführungsform das Griffteil 3 gegenüber dem anderen Griffteil 4 verschwenkbar an der Handhabe 5 gelagert ist.

Am distalen Ende des Schaftes 2 ist ein Werkzeug 6 angeordnet, welches beim dargestellten Ausführungsbeispiel aus einem starr mit dem Schaft 2 verbundenen Maulteil 6a sowie einem um eine Schwenkachse 7 verschwenkbaren Maulteil 6b besteht. Zum öffnen und Schließen der Maulteile 6a und 6b des Werkzeugs 6 über die Betätigung des verschwenkbaren Griffteils 3 der Handhabe 5 stehen das verschwenkbare Griffteile 3 und das verschwenkbare Maulteil 6b über ein im hohlen Schaft 2 gelagertes Zug-/Druckelement 8 miteinander in Wirkverbindung.

Wie aus den Abbildungen Fig. 1, 2, 4, 6 und 8 ersichtlich, sind die Maulteile 6a und 6b des Werkzeugs 6 nicht nur so ausgebildet, dass das Maulteil 6b gegenüber dem Maulteil 6a verschwenkbar ist, sondern zusätzlich auch das Maulteil 6a oder ein Teilbereich 6c des Maulteils 6a in Längsrichtung des Schaftes 2 gegenüber dem Maulteil 6b verschiebbar ausgebildet ist.

Alternativ zu der dargestellten Ausbildung der Maulteile 6a und 6b des Werkzeugs 6 ist es selbstverständlich auch möglich, dass das obere Maulteil 6b starr, das heißt nicht verschwenkbar ausgebildet ist und das untere Maulteil 6a verschwenkbar ausgebildet ist. Ebenso können das Maulteil 6b oder ein Teilbereich des Maulteils 6b gegenüber dem Maulteil 6a in Längsrichtung des Schaftes 2 verschiebbar ausgebildet sein. Auch die Relativverschiebbarkeit beider Maulteile 6a und 6b zueinander ist eine mögliche Konstruktionsvariante.

Durch diese Ausgestaltung der Maulteile 6a und 6b des Werkzeugs 6, wonach zumindest ein Teilbereich 6c mindestens eines Maulteils 6a oder 6b des Werkzeugs 6 in Längsrichtung des Schaftes 2 relativ zu dem anderen Maulteil 6b oder 6a des Werkzeugs 6 verschiebbar ausgebildet ist, ist es möglich, die Lage einer zwischen den Maulteilen 6a und 6b gehaltenen chirurgischen Nadel 9 ohne Zuhilfenahme eines zusätzlichen Werkzeugs oder manuelles Ergreifen auszurichten, da das Verschieben der Maulteile 6a und 6b relativ zueinander ein Verdrehen der Nadel 9 um ihre Längsachse bewirkt.

Um den Verschleiß der Greiffläche des verschiebbaren Maulteils 6a oder des verschiebbaren Teilbereichs 6c im Kontaktbereich mit der chirurgischen Nadel 9 zu minimieren, besteht zumindest der zur Aufnahme einer zu haltenden chirurgischen Nadel 9 dienende Teilbereich der Greiffläche mindestens eines Maulteils 6a, 6b aus einem Hartmetall.

Das Verstellen des verschiebbaren Maulteils 6a oder des verschiebbaren Teilbereichs 6c des Maulteils 6a erfolgt über einen an der Handhabe 5 angeordneten Antrieb 10, der vorzugsweise als um die Längsachse des Schaftes 2 verdrehbares Stellrad 11 ausgebildet ist.

Das verschiebbare Maulteil 6a oder der verschiebbare Teilbereich 6c des Maulteils 6) und der Antrieb 10 stehen über ein Betätigungselement 12 miteinander in Wirkverbindung, das die Rotationsbewegung des Stellrads 11 in eine Längsbewegung des verschiebbaren Maulteils 6a oder des verschiebbaren Teilbereichs 6c des Maulteils 6a überführt.

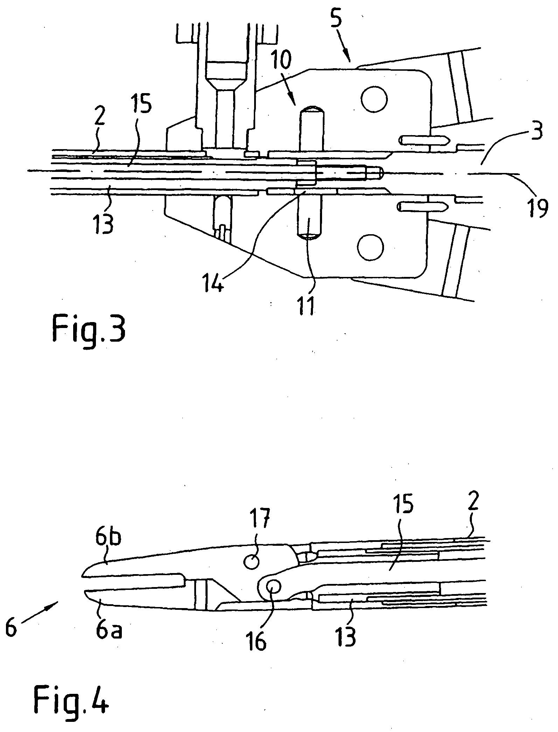

Die in den Abbildungen Fig. 3 bis 8 dargestellten Ausführungsformen zur Ausbildung eines medizinischen Nadelhalters 1 unterscheiden sich im Wesentlichen dadurch, wie das Verschwenken des verschwenkbaren Maulteils 6b über den verschwenkbaren Griffteil 3 der Handhabe 5 und das Verstellen des verschiebbaren Maulteils 6a oder des verschiebbaren Teilbereichs 6c des Maulteils 6a über den Antrieb 10 erfolgt.

Bei der in den Abbildungen Fig. 3 und 4 dargestellten ersten Ausführungsform des medizinischen Nadelhalters 1 sind das Betätigungselement 12 und das Zug-/Druckelement 8 koaxial zueinander im Schaft 2 angeordnet.

Wie aus Fig. 3 ersichtlich, ist das Betätigungselement 12 bei dieser Ausführungsform als im Schaft 2 angeordnetes Hohlrohr 13 ausgebildet ist, wobei das Stellrad 11 und das Hohlrohr 13 über ein Gewinde 14 so miteinander in Wirkverbindung stehen, dass die Rotationsbewegung des Stellrads 11 in eine rein axiale Verlagerung des Hohlrohres 13 umgesetzt wird.

Das Zug-/Druckelement 8 zur Betätigung des verschwenkbaren Maulteils 6b über den verschwenkbaren Griffteil 3 ist bei dieser Ausführungsform als axial verschiebbar in dem Hohlrohr 13 angeordnete Zug-/Druckstange 15 ausgebildet, die proximalseitig kraftschlüssig mit dem verschwenkbaren Griffteil 3 der Handhabe 5 gekoppelt ist.

Die Abbildung Fig. 4 zeigt das distalseitige Ende des medizinischen Nadelhalters 1 gemäß der zuvor beschriebenen ersten Ausführungsform. Wie aus der Abbildung ersichtlich, ist die Zug-/Druckstange 15 über einen Anlenkpunkt 16 so am verschwenkbaren Maulteil 6b gelagert, dass das verschwenkbare Maulteil 6b beim axialen Verschieben der Zug-/Druckstange 15 nach distal um eine Schwenkachse 17 in die in Fig. 1 gestrichelt dargestellte geöffnete Position verschwenkt wird. Umgekehrt bewirkt das axiale Zurückziehen der Zug-/Druckstange 15 nach proximal das Überführen des verschwenkbaren Maulteils 6b in die geschlossene Position.

Gemäß der dargestellten ersten Ausführungsform bildet das verschiebbare Maulteil 6a das distale Ende des Hohlrohres 13, so dass das Verdrehen des Stellrades 11 durch Umsetzung über das Gewinde 14 eine direkte Axialverlagerung des verschiebbaren Maulteils 6a bewirkt. Alternativ zu der dargestellten Ausführungsform ist es auch möglich, dass das distale. Ende des Hohlrohres 13 als verschiebbarer Teilbereich 6c des Maulteils 6a ausgebildet ist.

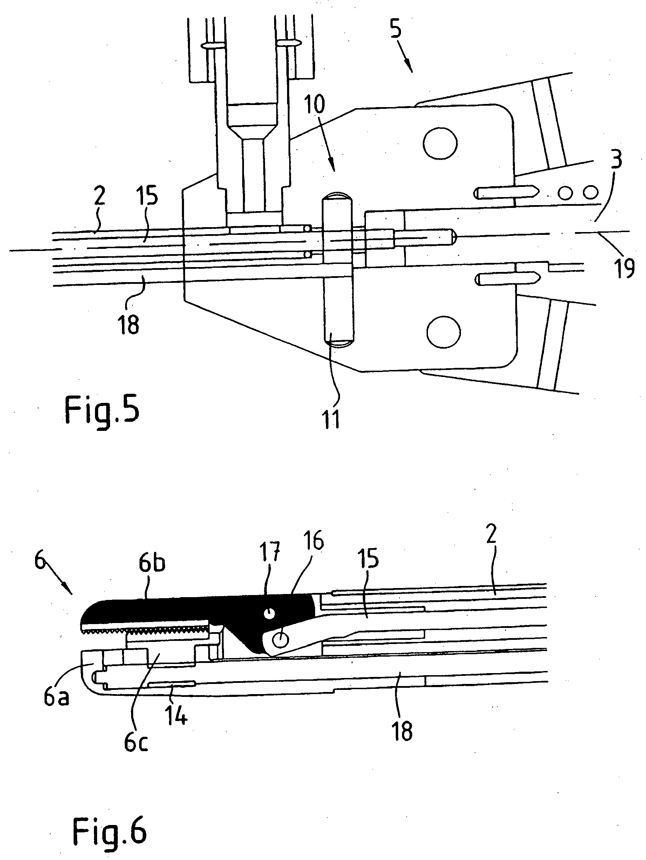

Bei den in den Abbildungen Fig. 5 bis 8 dargestellten erfindungsgemäßen Ausführungsformen zur Ausbildung der Relativverschiebbarkeit der Maulteile 6a und 6b des Werkzeugs 6 ist nicht das gesamte Maulteil 6a in Längsrichtung des Schaftes 2 verschiebbar ausgebildet, sondern nur der zur klemmenden Aufnahme der Nadel 9 dienende Teilbereich 6c des Maulteils 6a.

Bei der in den Abbildungen Fig. 5 und 6 dargestellten zweiten Ausführungsform des medizinischen Nadelhalters 1 sind das Betätigungselement 12 und das Zug-/Druckelement 8 parallel nebeneinander im Schaft 2 angeordnet.

Wie aus Fig. 5 ersichtlich, ist das Betätigungselement 12 bei dieser Ausführungsform als im Schaft 2 angeordnete Torsionsstange 18 ausgebildet ist, wobei das Stellrad 11 und die Torsionsstange 18 kraftschlüssig so miteinander verbunden sind, dass die Rotationsbewegung des Stellrads 11 direkt in eine Rotation der Torsionsstange 18 um die Längsachse 19 des Schaftes 2 umgesetzt wird.

Das Zug-/Druckelement 8 zur Betätigung des verschwenkbaren Maulteils 6b über den verschwenkbaren Griffteil 3 ist bei dieser Ausführungsform als axial verschiebbar in dem Hohlrohr 13 angeordnete Zug-/Druckstange 15 ausgebildet, die proximalseitig kraftschlüssig mit dem verschwenkbaren Griffteil 3 der Handhabe 5 gekoppelt ist.

Die Abbildung Fig. 6 zeigt das distalseitige Ende des medizinischen Nadelhalters 1 gemäß der zuvor beschriebenen zweiten Ausführungsform. Wie aus der Abbildung ersichtlich, ist die Zug-/Druckstange 15 über einen Anlenkpunkt 16 so am verschwenkbaren Maulteil 6b gelagert, dass das verschwenkbare Maulteil 6b beim axialen Verschieben der Zug-/Druckstange 15 nach distal um eine Schwenkachse 17 in die in Fig. 1 gestrichelt dargestellte geöffnete Position verschwenkt wird. Umgekehrt bewirkt das axiale Zurückziehen der Zug-/Druckstange 15 nach proximal das Überführen des verschwenkbaren Maulteils 6b in die geschlossene Position.

Gemäß der dargestellten zweiten Ausführungsform ist bei dieser Ausgestaltungsform nicht das gesamte Maulteil 6a, sondern nur ein Teilbereich 6c des Maulteils 6a in Längsrichtung des Schaftes 2 verschiebbar ausgebildet. Um die durch das Verdrehen des Stellrades 11 bewirkte Rotationsbewegung der Torsionsstange 18 wieder in eine rein axiale Bewegung des verschiebbaren Teilbereichs 6c des Maulteils 6a umzusetzen, stehen das distale Ende der Torsionsstange 18 und der verschiebbare Teilbereich 6c des Maulteils 6a über ein Gewinde 14 miteinander in Wirkverbindung.

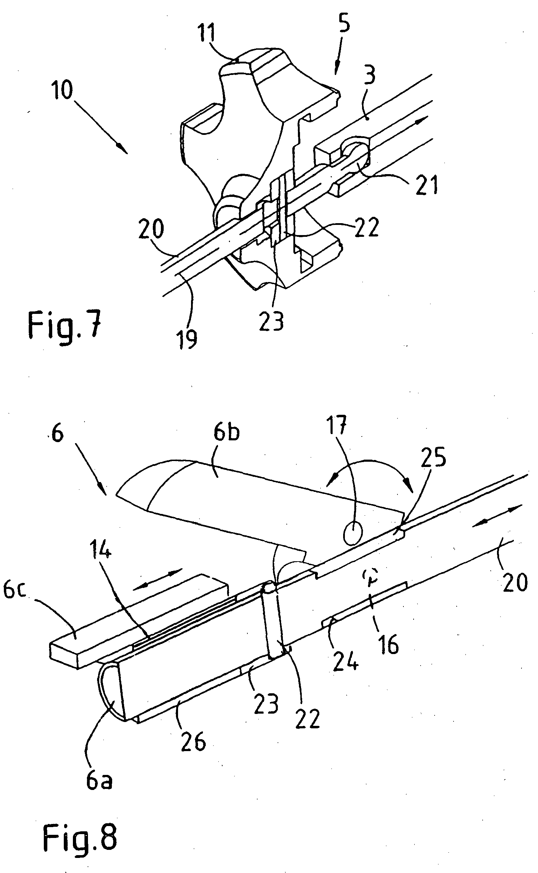

Bei der in den Abbildungen Fig. 7 und 8 dargestellten dritten Ausführungsform des medizinischen Nadelhalters 1 sind das Betätigungselement 12 und das Zug-/Druckelement 8 als im Schaft 2 angeordnetes einstückiges stangenförmiges Antriebselement 20 ausgebildet.

Wie aus Fig. 7 ersichtlich, ist zur Übertragung der Axialbewegung von dem verstellbar ausgebildeten Griffteil 3 der Handhabe 5 auf das verschwenkbare Maulteil 6b des Werkzeugs 6 das Antriebselement 20 proximalseitig über ein kugelförmiges Anschlusselement 21 am verstellbar ausgebildeten Griffteil 3 gelagert, wobei die kugelförmige Ausgestaltung des proximalen Endes des Antriebselements 20 eine frei drehbare Lagerung des Antriebselements 20 am verstellbaren Griffteil 3 bei gleichzeitiger Spielfreiheit zur Übertragung der Axialbewegung ermöglicht.

Wie weiterhin aus Fig. 7 ersichtlich, sind das Stellrad 11 und die Torsionsstange 18 kraftschlüssig so miteinander verbunden sind, dass die Rotationsbewegung des Stellrads 11 direkt in eine Rotation des Antriebselements 20 um die Längsachse 19 des Schaftes 2 umgesetzt wird, wobei zur Ausbildung der kraftschlüssigen Verbindungen des Stellrads 11 mit dem Antriebselements 20 ein Mitnahmestift 22 im Antriebselement 20 angeordnet sind, der in das Stellrad 11 eingreift.

Um die Axialbewegung und die Rotationsbewegung des Antriebselements 20 voneinander zu entkoppeln ist im Bereich der kraftschlüssigen Verbindung des Antriebselements 20 mit dem Stellrad 11 eine nutenförmige Ausgleichsführung 23 im Stellrad 11 ausgebildet, so dass das Antriebselement 20 über den verschwenkbaren Griffteil 3 in Axialrichtung verschoben werden kann, ohne durch die kraftschlüssige Kopplung zwischen dem Stellrad 11 und dem Antriebselement 20 blockiert zu werden.

Die Abbildung Fig. 8 zeigt das distalseitige Ende des medizinischen Nadelhalters 1 gemäß der zuvor beschriebenen dritten Ausführungsform. Wie aus der Abbildung ersichtlich, ist zur Übertragung der Axialbewegung von dem verstellbar ausgebildeten Griffteil 3 der Handhabe 5 auf das verschwenkbare Maulteil 6b des Werkzeugs 6 das Antriebselement 20 distalseitig im Bereich der Lagerung am verschwenkbaren Maulteil 6a in der äußeren Mantelfläche des Antriebselements 20 eine umlaufende Nut 24 ausgebildet ist, in der ein um die Längsachse des Antriebselements 20 drehbarer Ring 25 angeordnet ist, an dem das verschwenkbare Maulteil 6a über einen Anlenkpunkt 16 so gelagert ist, dass das verschwenkbare Maulteil 6b beim axialen Verschieben des Antriebselements 20 nach distal um eine Schwenkachse 17 in die in Fig. 1 gestrichelt dargestellte geöffnete Position verschwenkt wird. Umgekehrt bewirkt das axiale Zurückziehen des Antriebselements 20 nach proximal das Überführen des verschwenkbaren Maulteils 6b in die geschlossene Position.

Gemäß der dargestellten dritten Ausführungsform ist auch bei dieser Ausgestaltungsform nicht das gesamte Maulteil 6a, sondern nur ein Teilbereich 6c des Maulteils 6a in Längsrichtung des Schaftes 2 verschiebbar ausgebildet.

Zur Übertragung der Rotationsbewegung des Stellrads 11 in eine Längsbewegung des verschiebbaren Teilbereichs 6c des Maulteils 6a steht das Antriebselement 20 distalseitig im Bereich der Lagerung am verschiebbaren Teilbereichs 6c des Maulteils 6a kraftschlüssig mit einer koaxial auf dem distalen Ende des Antriebselements 20 gelagerten Hülse 26 in Wirkverbindung, die über ein Gewinde 14 mit dem verschiebbaren Teilbereich 6c des Maulteils 6a verbunden ist, wobei zur Ausbildung der kraftschlüssigen Verbindungen des Stellrads 11 mit dem Antriebselements 20 ein Mitnahmestift 22 im Antriebselement 20 angeordnet sind, der in die Hülse 26 eingreift.

Um die Axialbewegung und die Rotationsbewegung des Antriebselements 20 voneinander zu entkoppeln ist im Bereich der kraftschlüssigen Verbindung des Antriebselements 20 mit der Hülse 26 eine nutenförmige Ausgleichsführung 23 in der Hülse 26 ausgebildet, so dass das Antriebselement 20 über den verschwenkbaren Griffteil 3 in Axialrichtung verschoben werden kann, ohne durch die kraftschlüssige Kopplung zwischen der Hülse 26 und dem Antriebselement 20 blockiert zu werden.

Die Handhabung des zuvor beschriebenen und in den Abbildungen Fig. 1 bis 8 dargestellten medizinischen Nadelhalters 1 geschieht wie folgt:

Ausgehend von der in Fig. 1 gestrichelt dargestellten geöffneten Stellung des Werkzeugs 6 wird der Nadelhalter 1 vom Bediener so platziert, dass die chirurgische Nadel 9 zwischen den Maulteilen 6a und 6b des Werkzeugs 6 zu liegen kommt. Anschließend drückt der Bediener die Griffteile 3 und 4 der Handhabe 5 zusammen, so dass das verschwenkbare Maulteil 6b geschlossen und die Nadel zwischen den beiden Maulteilen 6a und 6b klemmend gehalten wird.

Die in der Regel sichelförmig gebogene Nadel 9 muss zum Vernähen einer Operationsstelle so im Nadelhalter 1 ausgerichtet werden, dass der Operateur die Nadel 9 im richtigen Winkel in das zu vernähende Gewebe einstechen kann.

Das Ausrichten der zwischen den Maulteilen 6a und 6b gehaltenen Nadel 9 geschieht bei den beschriebenen Nadelhaltern 1 dadurch, dass durch Betätigen des an der Handhabe 5 angeordneten Antriebs 10 entweder das gesamte Maulteil 6a oder nur ein Teilbereich 6c des Maulteils 6a gegenüber dem anderen Maulteil 6b in Längsrichtung des Schaftes 2 verschoben wird, was eine Rotation der Nadel 9 um ihre Längsachse bewirkt.

Um einerseits einen stets sicheren Halt der chirurgischen Nadel 9 im Werkzeug 6 zu gewährleisten und andererseits den Bediener zu entlasten, so dass dieser die Griffteile 3 und 4 der Handhabe 5 nicht fortwährend betätigen muss, sind die Griffteile 3 und 4 der Handhabe 5 über einen Arretiermechanismus 27 in ihrer jeweiligen Position zueinander fixierbar sind, wie dies den Abbildungen Fig. 1 und 2 zu entnehmen ist.

Mittels dieses Arretiermechanismus 27 ist es möglich, die Nadel in einem ersten Schritt nur so zwischen den Maulteilen 6a und 6b festzulegen, dass diese zum Ausrichten noch verdrehbar ist und erst nachfolgend das endgültige Klemmen der Nadel 9 in der richtigen Lage erfolgt.

Um nach dem Loslassen der chirurgischen Nadel 9 dem verschiebbaren Maulteil 6a bzw. dem verschiebbaren Teilbereich 6c des Maulteils 6a wieder den gleichen Bewegungsspielraum zum erneuten Ergreifen der Nadel 9 zu ermöglichen, ist das verschiebbare Maulteil 6a bzw. der verschiebbare Teilbereich 6c des Maulteils 6a über ein nicht dargestelltes Federelement in eine Ausgangsposition vorgespannt.

Ein solchermaßen ausgebildeter medizinischer Nadelhalter 1 zeichnet sich dadurch aus, dass der eine einfache Positionierung der Nadel 9 im Werkzeug 6 ohne Zuhilfenahme eines weiteren Instruments ermöglicht.

**Bezugszeichenliste**

| | | | |
|---|---|---|---|
| 1 | medizinischer Nadelhalter | 15 | Zug-/Druckstange |
| | | | |
| 2 | Schaft | 16 | Anlenkpunkt |
| | | | |
| 3 | Griffteil (verschwenkbar) | 17 | Schwenkachse |
| | | | |
| 4 | Griffteil (starr) | 18 | Torsionsstange |
| | | | |
| 5 | Handhabe | 19 | Längsachse |
| | | | |
| 6 | Werkzeug | 20 | Antriebselement |
| 6a | starres Maulteil | 21 | Anschlusselement |
| | | | |
| 6b | verschwenkbares Maulteil | 22 | Mitnahmestift |
| | | | |
| 6c | Teilbereich (verschiebbar) | 23 | Ausgleichsführung |
| | | | |
| 7 | Schwenkachse | 24 | Nut |
| | | | |
| 8 | Zug-/Druckelement | 25 | Ring |
| | | | |
| 9 | Nadel | 26 | Hülse |
| | | | |
| 10 | Antrieb | 27 | Arretiermechanismus |
| | | | |
| 11 | Stellrad | | |
| | | | |
| 12 | Betätigungselement | | |
| | | | |
| 13 | Hohlrohr | | |
| | | | |
| 14 | Gewinde | | |

## Patentansprüche

1. Medizinischer Nadelhalter mit einem Schaft (2), mit einem am distalen Ende des Schaftes (2) angeordneten, aus zwei Maulteilen (6a, 6b) bestehenden Werkzeug (6), wobei ein Maulteil (6b) des Werkzeugs (6) gegenüber dem anderen Maulteil (6a) des Werkzeugs (6) verschwenkbar ausgebildet ist, sowie mit einer am proximalen Ende des Schaftes (2) angeordneten, mindestens zwei Griffteile (3, 4) aufweisenden Handhabe (5), wobei das verschwenkbare Maulteil (6b) des Werkzeugs (6) über ein verstellbar ausgebildetes Griffteil (3) der Handhabe (5) betätigbar ist, wobei ausschließlich ein, zur Klemmenden Aufnahme der Nadel (9) dienender Teilbereich (6c) des nicht verschwenkbaren Maulteils (6a) des Werkzeugs (6) in Längsrichtung des Schaftes (2) relativ zu dem anderen Maulteil (6b) des Werkzeugs (6) verschiebbar ausgebildet ist und dass das Verschieben in Längsrichtung des Schaftes (2) über einen an der Handhabe (5) angeordneten Antrieb (10) erfolgt.

2. Medizinischer Nadelhalter nach Anspruch 1, **dadurch gekennzeichnet, dass** der Antrieb (10) als Stellrad (11) ausgebildet ist.

3. Medizinischer Nadelhalter nach Anspruch 2, **dadurch gekennzeichnet, dass** der verschiebbare Teilbereich (6c) des Maulteils (6a) und der Antrieb (10) über ein Betätigungselement (12) miteinander in Wirkverbindung stehen, das die Rotationsbewegung des Stellrads (11) in eine Längsbewegung des verschiebbaren Teilbereichs (6c) des Maulteils (6a) überführt.

4. Medizinischer Nadelhalter nach einem der Ansprüche 1 bis 3, **dadurch gekennzeichnet, dass** das verschwenkbare Maulteil (6b) des Werkzeugs (6) und das verstellbar ausgebildete Griffteil (3) der Handhabe (5) über ein Zug-/Druckelement (8) miteinander in Wirkverbindung stehen.

5. Medizinischer Nadelhalter nach den Ansprüchen 3 und 4, **dadurch gekennzeichnet, dass** das Betätigungselement (12) und das Zug-/Druckelement (8) koaxial zueinander im Schaft (2) angeordnet sind.

6. Medizinischer Nadelhalter nach Anspruch 5, **dadurch gekennzeichnet, dass** das Betätigungselement (12) als im Schaft (2) angeordnetes Hohlrohr (13) ausgebildet ist, wobei das Stellrad (11) und das Hohlrohr (13) über ein Gewinde (14) miteinander in Wirkverbindung stehen und wobei der verschiebbare Teilbereich (6c) des Maulteils (6a) das distale Ende des Hohlrohres (13) bildet.

7. Medizinischer Nadelhalter nach Anspruch 6, **dadurch gekennzeichnet, dass** das Zug-/Druckelement (8) als axial verschiebbar in dem Hohlrohr (13) angeordnete Zug-/Druckstange (15) ausgebildet ist.

8. Medizinischer Nadelhalter nach den Ansprüchen 3 und 4, **dadurch gekennzeichnet, dass** das Betätigungselement (12) und das Zug-/Druckelement (8) parallel nebeneinander im Schaft (2) angeordnet sind.

9. Medizinisches Nadelhalter nach Anspruch 8, **dadurch gekennzeichnet, dass** das Betätigungselement (12) als im Schaft (2) angeordnete Torsionsstange (18) ausgebildet ist, wobei das Stellrad (11) und die Torsionsstange (18) kraftschlüssig miteinander verbunden sind und wobei der verschiebbare Teilbereich (6c) des Maulteils (6a) und das distale Ende der Torsionsstange (18) über ein Gewinde (14) miteinander in Wirkverbindung stehen.

10. Medizinischer Nadelhalter nach Anspruch 9, **dadurch gekennzeichnet, dass** das Zug-/Druckelement (8) als im Schaft (2) angeordnete Zug-/Druckstange (15) ausgebildet ist.

11. Medizinischer Nadelhalter nach den Ansprüchen 3 und 4, **dadurch gekennzeichnet, dass** das Betätigungselement (12) und das Zug-/Druckelement (8) als im Schaft (2) angeordnetes einstückiges stangenförmiges Antriebselement (20) ausgebildet sind.

12. Medizinischer Nadelhalter nach Anspruch 11 **dadurch gekennzeichnet, dass** zur Übertragung der Axialbewegung von dem verstellbar ausgebildeten Griffteil (3) der Handhabe (5) auf das verschwenkbare Maulteil (6b) des Werkzeugs (6) das Antriebselement (20) proximalseitig über ein kugelförmiges Anschlusselement (21) am verstellbar ausgebildeten Griffteil (3) gelagert ist und distalseitig im Bereich der Lagerung am verschwenkbaren Maulteil (6a) in der äußeren Mantelfläche des Antriebselements (20) eine umlaufende Nut (24) ausgebildet ist, in der ein um die Längsachse des Antriebselements (20) drehbarer Ring (25) angeordnet ist, an dem das verschwenkbare Maulteil (6a) gelagert ist.

13. Medizinischer Nadelhalter nach Anspruch 11 oder 12 **dadurch gekennzeichnet, dass** das Antriebselement (20) zur Übertragung der Rotationsbewegung des Stellrads (11) in eine Längsbewegung des verschiebbaren Teilbereichs (6c) des Maulteils (6a) proximalseitig kraftschlüssig mit dem Stellrad (11) verbunden ist und distalseitig im Bereich der Lagerung am verschiebbaren Teilbereichs (6c) des Maulteils (6a) kraftschlüssig mit einer koaxial auf dem distalen Ende des Antriebselements (20) gelagerten Hülse (26) in Wirkverbindung steht, die über ein Gewinde (14) mit dem verschiebbaren Teilbereich (6c) des Maulteils (6a) verbunden ist.

14. Medizinischer Nadelhalter nach Anspruch 13 **dadurch gekennzeichnet, dass** in den Bereichen der kraftschlüssigen Verbindungen des Antriebselements (20) mit dem Stellrad (11) sowie des Antriebselements (20) mit der Hülse (26) Ausgleichsführungen (23) ausgebildet sind, um die Axialbewegung und die Rotationsbewegung des Antriebselements (20) voneinander zu entkoppeln.

15. Medizinischer Nadelhalter nach Anspruch 13 oder 14 **dadurch gekennzeichnet, dass** zur Ausbildung der kraftschlüssigen Verbindungen des Antriebselements (20) mit dem Stellrad (11) sowie des Antriebselements (20) mit der Hülse (26) Mitnahmestifte (22) im Antriebselement (20) angeordnet sind, die in das jeweils andere Bauteil Stellrad (11) und Hülse (26) eingreifen.

16. Medizinischer Nadelhalter nach Anspruch 15 **dadurch gekennzeichnet, dass** die Ausgleichsführungen (23) als im Stellrad (11) und in der Hülse (26) angeordnete, in Axialrichtung verlaufende Nuten ausgebildet sind, in denen die Mitnahmestifte (22) gelagert sind.

## Claims

1. A medical needle holder comprising a shaft (2), a tool (6) arranged at the distal end of the shaft (2) and comprising two jaw parts (6a, 6b), one jaw part (6b) of the tool (6) being embodied as pivotable in relation to the other jaw part (6a) of the tool (6), and a handle (5) arranged at the proximal end of the shaft (2) and having at least two grip parts (3, 4), wherein the pivotable jaw part (6b) of the tool (6) can be manipulated via an adjustable grip part (3) of the handle (5), wherein
only one portion (6c) of the non-pivotable jaw part (6a) of the tool (6), which portion serves to receive and clamp the needle (9), is embodied as displaceable in the longitudinal direction of the shaft (2) relative to the other jaw part (6b) of the tool (6), and wherein the displacement in the longitudinal direction of the shaft (2) is effected by means of an actuating mechanism (10) arranged on the handle (5).

2. The medical needle holder according to claim 1, **characterized in that** the actuating mechanism (10) is embodied as an adjusting wheel (11).

3. The medical needle holder according to claim 2, **characterized in that** the displaceable portion (6c) of the jaw part (6a) and the actuating mechanism (10) are operatively connected to one another via an actuating element (12), which converts the rotational movement of the adjusting wheel (11) into a longitudinal movement of the displaceable portion (6c) of the jaw part (6a).

4. The medical needle holder according to any one of claims 1 to 3, **characterized in that** the pivotable jaw part (6b) of the tool (6) and the adjustable grip part (3) of the handle (5) are operatively connected to one another via a push/pull element (8).

5. The medical needle holder according to claims 3 and 4, **characterized in that** the actuating element (12) and the push/pull element (8) are arranged coaxially with one another in the shaft (2).

6. The medical needle holder according to claim 5, **characterized in that** the actuating element (12) is embodied as a hollow tube (13) arranged in the shaft (2), wherein the adjusting wheel (11) and the hollow tube (13) are operatively connected to one another via a thread (14) and wherein the displaceable portion (6c) of the jaw part (6a) forms the distal end of the hollow tube (13).

7. The medical needle holder according to claim 6, **characterized in that** the push/pull element (8) is embodied as a push/pull rod (15) arranged axially displaceably in the hollow tube (13).

8. The medical needle holder according to claims 3 and 4, **characterized in that** the actuating element (12) and the push/pull element (8) are arranged side by side and parallel in the shaft (2).

9. The medical needle holder according to claim 8, **characterized in that** the actuating element (12) is embodied as a torsion rod (18) arranged in the shaft (2), wherein the adjusting wheel (11) and the torsion rod (18) are frictionally connected to one another, and wherein the displaceable portion (6c) of the jaw part (6a) and the distal end of the torsion rod (18) are operatively connected to one another via a thread (14).

10. The medical needle holder according to claim 9, **characterized in that** the push/pull element (8) is embodied as a push/pull rod (15) arranged in the shaft (2).

11. The medical needle holder according to claims 3 and 4, **characterized in that** the actuating element (12) and the push/pull element (8) are embodied as an integral rod-shaped actuating element (20) arranged in the shaft (2).

12. The medical needle holder according to claim 11, **characterized in that** in order to transmit the axial movement from the adjustable grip part (3) of the handle (5) to the pivotable jaw part (6b) of the tool (6), the actuating element (20) is mounted at its proximal end on the adjustable grip part (3) via a spherical connection element (21), and at its distal end, in the region where it is mounted on the pivotable jaw part (6b), a circumferential groove (24) is formed in the circumferential surface of the actuating element (20), in which a ring (25) is arranged, which is rotatable about the longitudinal axis of the actuating element (20) and on which the pivotable jaw part (6b) is mounted.

13. The medical needle holder according to claim 11 or 12, **characterized in that** in order to convert the rotational movement of the adjusting wheel (11) into a longitudinal movement of the displaceable portion (6c) of the jaw part (6a), the actuating element (20) is frictionally connected at its proximal end to the adjusting wheel (11), and at its distal end, in the region where it is mounted on the displaceable portion (6c) of the jaw part (6a), the actuating element is operatively frictionally connected to a sleeve (26), which is mounted coaxially on the distal end of the actuating element (20) and which is connected to the displaceable portion (6c) of the jaw part (6a) via a thread (14).

14. The medical needle holder according to claim 13, **characterized in that** in the regions of the frictional connections between the actuating element (20) and the adjusting wheel (11) and between the actuating element (20) and the sleeve (26), leveling guides (23) are provided for decoupling the axial movement of the actuating element (20) from the rotational movement thereof.

15. The medical needle holder according to claim 13 or 14, **characterized in that**, to form the frictional connections between the actuating element (20) and the adjusting wheel (11) and between the actuating element (20) and the sleeve (26), locking pins (22) are arranged in the actuating element (20), each pin engaging in the respective other component, either adjusting wheel (11) or sleeve (26).

16. The medical needle holder according to claim 15, **characterized in that** the leveling guides (23) are embodied as grooves arranged in the adjusting wheel (11) and in the sleeve (26) and extending in the axial direction, with the locking pins (22) being supported in said grooves.

## Revendications

1. Porte-aiguille médical comprenant un corps allongé (2), avec un outil (6) constitué de deux pièces de mâchoire (6a, 6b), agencé à l'extrémité distale du corps allongé (2), une pièce de mâchoire (6b) de l'outil (6) étant d'une configuration pivotante par rapport à l'autre pièce de mâchoire (6a) de l'outil (6), ainsi qu'avec une poignée (5) présentant au moins deux pièces de poignée (3, 4) et agencée à l'extrémité proximale du corps allongé (2), la pièce de mâchoire pivotante (6b) de l'outil (6) pouvant être actionnée par l'intermédiaire d'une pièce de poignée (3) déplaçable de la poignée (5), porte-aiguille dans lequel exclusivement un secteur partiel (6c) de la pièce de mâchoire (6a) de l'outil (6), qui sert à assurer la réception par serrage de l'aiguille (9), est réalisé de manière à pouvoir coulisser, par rapport à l'autre pièce de mâchoire (6b) de l'outil (6), dans la direction longitudinale du corps allongé (2), et dans lequel le coulissement dans la direction longitudinale du corps allongé (2) s'effectue par l'intermédiaire d'un entraînement (10) agencé dans la poignée (5).

2. Porte-aiguille médical selon la revendication 1, **caractérisé en ce que** l'entraînement (10) est réalisé en tant que molette de réglage (11).

3. Porte-aiguille médical selon la revendication 2, **caractérisé en ce que** le secteur partiel coulissant (6c) de la pièce de mâchoire (6a) et l'entraînement (10) sont en liaison d'interaction par l'intermédiaire d'un élément d'actionnement (12), qui convertit le mouvement de rotation de la molette de réglage (11) en un mouvement longitudinal du secteur partiel (6c) de la pièce de mâchoire (6a).

4. Porte-aiguille médical selon l'une des revendications 1 à 3, **caractérisé en ce que** la pièce de mâchoire (6b) pivotante de l'outil (6) et la pièce de poignée (3) déplaçable de la poignée (5) sont en liaison d'interaction par l'intermédiaire d'un élément de traction/compression (8).

5. Porte-aiguille médical selon les revendications 3 et 4, **caractérisé en ce que** l'élément d'actionnement (12) et l'élément de traction/compression (8) sont agencés de manière mutuellement coaxiale dans le corps allongé (2).

6. Porte-aiguille médical selon la revendication 5, **caractérisé en ce que** l'élément d'actionnement (12) est réalisé sous forme de tube creux (13) agencé dans le corps allongé (2), la molette de réglage (11) et le tube creux (13) étant en liaison d'interaction mutuelle par l'intermédiaire d'un filetage (14), et le secteur partiel (6c) coulissant de la pièce de mâchoire (6a) formant l'extrémité distale du tube creux (13).

7. Porte-aiguille médical selon la revendication 6, **caractérisé en ce que** l'élément de traction/compression (8) est réalisé en tant que tige de traction/compression (15) agencée de manière axialement coulissante dans le tube creux (13).

8. Porte-aiguille médical selon les revendications 3 et 4, **caractérisé en ce que** l'élément d'actionnement (12) et l'élément de traction/compression (8) sont agencés parallèlement côte à côte dans le corps allongé (2).

9. Porte-aiguille médical selon la revendication 8, **caractérisé en ce que** l'élément d'actionnement (12) est réalisé en tant que tige de torsion (18) agencée dans le corps allongé (2), la molette de réglage (11) et la tige de torsion (18) étant reliées mutuellement par une liaison de transmission de force, et le secteur partiel (6c) coulissant de la pièce de mâchoire (6a) et l'extrémité distale de la tige de torsion (18) étant en liaison d'interaction mutuelle par l'intermédiaire d'un filetage (14).

10. Porte-aiguille médical selon la revendication 9, **caractérisé en ce que** l'élément de traction/compression (8) est réalisé en tant que tige de traction/compression (15) agencée dans le corps allongé (2).

11. Porte-aiguille médical selon les revendications 3 et 4, **caractérisé en ce que** l'élément d'actionnement (12) et l'élément de traction/compression (8) sont réalisés en tant qu'élément d'entraînement (20) d'un seul tenant en forme de tige, agencé dans le corps allongé (2).

12. Porte-aiguille médical selon la revendication 11, **caractérisé en ce que** pour la transmission du mouvement axial de la pièce de poignée déplaçable (3) de la poignée (5) à la pièce de mâchoire pivotante (6b) de l'outil (6), l'élément d'entraînement (20) est monté, côté proximal, par l'intermédiaire d'un élément de raccordement (21) en forme de rotule, sur la pièce de poignée (3) déplaçable, et du côté distal, dans la zone du montage sur la pièce de mâchoire pivotante (6a), dans la surface périphérique extérieure de l'élément d'entraînement (20) est formée une rainure périphérique (24) dans laquelle est agencé un anneau (25) pouvant tourner autour de l'axe longitudinal de l'élément d'entraînement (20) et sur lequel est monté la pièce de mâchoire pivotante (6a).

13. Porte-aiguille médical selon la revendication 11 ou la revendication 12, **caractérisé en ce que** l'élément d'entraînement (20), pour convertir le mouvement de rotation de la molette de réglage (11) en un mouvement longitudinal du secteur partiel (6c) coulissant de la pièce de mâchoire (6a), est relié, côté proximal, par une liaison de transmission de force, avec la molette de réglage (11), et, côté distal, dans la zone du montage sur le secteur partiel (6c) coulissant de la pièce de mâchoire (6a), est en liaison d'interaction de transmission de force avec une douille (26), qui est montée coaxialement sur l'extrémité distale de l'élément d'entraînement (20), et est reliée, par un filetage (14), au secteur partiel (6c) coulissant de la pièce de mâchoire (6a).

14. Porte-aiguille médical selon la revendication 13, **caractérisé en ce que** dans les zones des liaisons de transmission de force de l'élément d'entraînement (20) avec la molette de réglage (11) ainsi que de l'élément d'entraînement (20) avec la douille (26), sont formés des guidages de compensation (23) en vue de découpler l'un de l'autre le mouvement axial et le mouvement de rotation de l'élément d'entraînement (20).

15. Porte-aiguille médical selon la revendication 13 ou la revendication 14, **caractérisé en ce que** pour réaliser les liaisons de transmission de force de l'élément d'entraînement (20) avec la molette de réglage (11) ainsi que de l'élément d'entraînement (20) avec la douille (26), des goupilles d'entraînement (22) sont agencées dans l'élément d'entraînement (20), et s'engagent dans l'autre pièce respective, à savoir la molette de réglage (11) et la douille (26).

16. Porte-aiguille médical selon la revendication 15, **caractérisé en ce que** les guidages de compensation (23) sont réalisés en tant que rainures, qui s'étendent dans la direction axiale et sont agencées dans la molette de réglage (11) et la douille (26), et dans lesquelles sont montées les goupilles d'entraînement (22).
